# FASCICULE DE BREVET EUROPEEN

(11) **EP 2 663 846 B1**
(45) Date de publication et mention de la délivrance du brevet: **28.02.2018**
(21) Numéro de dépôt: 12705321.3
(22) Date de dépôt: 12.01.2012
(51) Int. Cl.: G01L 1/16, A61B 5/103

(54) **DISPOSITIF DE MESURE DE LA PRESSION A PARTIR D'UN OBJET SOUPLE, PLIABLE ET/OU EXTENSIBLE REALISE A PARTIR DE MATIERE TEXTILE COMPORTANT UN DISPOSITIF DE MESURE**
VORRICHTUNG ZUR MESSUNG DES VON EINEM AUS EINEM TEXTILMATERIAL HERGESTELLTEN FLEXIBLEN, BIEGSAMEN UND/ODER DEHNBAREN OBJEKTS AUSGEHENDEN DRUCKS MIT EINER MESSVORRICHTUNG
DEVICE FOR MEASURING PRESSURE FROM A FLEXIBLE, PLIABLE, AND/OR EXTENSIBLE OBJECT MADE FROM A TEXTILE MATERIAL COMPRISING A MEASUREMENT DEVICE

(30) Priorité: 13.01.2011 FR 1150283
(43) Date de publication de la demande: 20.11.2013
(73) Titulaire: Texisense, 71300 Monceau les Mines (FR)
(72) Inventeur: DIOT, Bruno, F-71640 Mercurey (FR); VUILLERME, Nicolas, F-73800 Francin (FR); PAYAN, Yohann, F-38580 Allevard (FR); LAVARENNE, Christophe, 71100 Saint-Remy (FR); CANNARD, Francis, 21700 Magny les Villers (FR)
(74) Mandataire: Cabinet Laurent & Charras
(86) Numéro de dépôt international: PCT/FR2012/050074
(87) Numéro de publication internationale: WO 2012/095608

(56) Documents cités:
- WO-A1-01/75778
- WO-A1-01/88935
- WO-A1-87/01574
- WO-A1-2005/096133
- WO-A1-2009/023937
- US-A1- 2002 194 934

## Description

### DOMAINE DE L'INVENTION

La présente invention concerne un dispositif de mesure de la pression à partir d'un objet souple ou pliable tel qu'un tissu par exemple, particulièrement adapté pour la mesure de pression afin de prévenir l'apparition de plaies de pression chez des personnes atteintes de maladies chroniques, d'une perte de mobilité ou de sensibilité par exemple.

### ARRIERE PLAN DE L'INVENTION

Il est bien connu que mesurer la pression, et de manière plus générale la distribution de forces sur une surface déterminée, peut être obtenue au moyen de capteurs de pression dits capacitifs. Lesdits capteurs de pression capacitifs comportent une ou plusieurs cellules capacitives qui sont positionnées de manière appropriée sur la surface de contact du capteur. Chaque cellule capacitive comprend une paire de panneaux obtenus dans un matériau électriquement conducteur et une couche d'un matériau isolant ou diélectrique positionnée entre les panneaux conducteurs.

La mesure de la pression agissant sur les cellules capacitives du capteur est obtenue en mesurant la variation de la capacitance des cellules capacitives produite par la variation de la distance entre les panneaux conducteurs sur lesquels une pression est exercée.

C'est le cas notamment de la demande de brevet européen EP 1211633 qui décrit un dispositif de mesure de la distribution de pression sur une surface.

On connaît également des dispositifs de mesure de la pression comportant des capteurs de pression dits résistifs.

C'est le cas notamment du brevet américain US 6,155,120 qui décrit un procédé et un appareil de mesure de la pression d'un pied au moyen de la variation de la piézorésistance. Ledit appareil comporte une matrice rectangulaire de capteurs de force piézorésistifs enfermés à l'intérieur d'une fine enveloppe polymère que l'on introduit à l'intérieur d'une chaussure, ou que l'on incorpore à une chaussette susceptible d'habiller un pied ou un sabot. La réalisation préférée de l'invention utilise des éléments piézorésistifs de détection de pressions ou de forces normales, qui comportent un réseau polymère tissé imprégné de particules conductrices en suspension à l'intérieur d'un véhicule élastomère, de préférence du caoutchouc silicone. La couche piézorésistive maillée est prise en sandwich entre une matrice de structures lamifiées constituées de bandes conductrices organisées en colonnes et en rangées, de préférence constituée d'un réseau en Nylon(R) imprégné de trajets métalliques imprimés. Dans une variation de la réalisation de base, chaque élément de détection des forces normales est bordé de paires d'éléments de détection des forces de cisaillement, disposées latéralement et longitudinalement, chacun des éléments de détection des forces de cisaillement comportant une paire de tampons piézorésistifs élastiques adjacents qui possèdent des surfaces latérales longitudinalement en contact. Ces tampons peuvent se déplacer par glissement, et lorsqu'ils sont forcés à un contact plus ou moins rapproché en réponse à des forces de cisaillement dirigées perpendiculairement à leur plan de contact tangent, la résistance électrique entre les tampons varie de manière préétablie en fonction des forces de cisaillement.

C'est également le cas de la demande de brevet international WO 87/01574 qui décrit un tapis de détection. Ledit tapis de détection se compose de moyens de support, d'une couche supérieure, d'une couche intermédiaire et d'une couche inférieure qui sont fixées l'une sur l'autre. La couche supérieure se compose d'un circuit imprimé souple sur la surface inférieure de laquelle des pistes conductrices sont déposées. La couche intermédiaire est constituée d'une feuille de caoutchouc conductrice et la couche inférieure est constituée d'un circuit imprimé rigide sur la surface supérieure duquel des pistes conductrices sont déposées.

On connaît également la demande de brevet américain US 2009/0128168 qui décrit des structures polymère conductrice multifonctionnelles et plus particulièrement l'utilisation de polymères conducteurs comme capteurs dans des systèmes de détection distribués, comme capteurs et éléments d'actionnement dans des dispositifs multifonctionnels, et pour des tissus multifonctionnels comprenant de tels polymères conducteurs pour contrôler l'humidité, la respiration, le rythme cardiaque, la pression sanguine, la température de la peau, le poids et les mouvements, dans un système de capteurs intégrés dans des vêtements, par exemple. Un tissu comprenant des fibres conductrices de polyaniline qui peuvent être utilisées aussi bien pour distribuer l'énergie dans un chauffage résistif que pour mesurer la température du tissu est décrit comme exemple d'un capteur multifonctionnel en tissu. Dans une variante d'exécution, il est décrit un tissu comportant des fibres de polyaniline pour la réalisation d'un capteur de pression, la pression étant déduite directement de la variation de la résistance des fibres de polyaniline.

Tous ces dispositifs comportant des capteurs capacitifs ou résistifs présentent les l'inconvénient de présenter une faible souplesse limitant considérablement leur champ d'application, ces dispositifs procurant un confort insuffisant pour être utilisés dans des vêtements par exemple. Par ailleurs, ces dispositifs présentent des coûts de fabrication élevés, dus à l'utilisation de moyens de production spécifiques, incompatibles avec une diffusion de grande série et la pression mesurée par les capteurs capacitifs dépend des phénomènes ambiants tels que la température et/ou l'humidité.

Afin de remédier à certains de ces inconvénients, on a déjà imaginé des capteurs de pression aptes à être utilisés dans des vêtements ou similaires. C'est le cas notamment des demandes de brevet WO 2005/096133 et WO 2009/023937 notamment.

Le document WO 2005/096133 décrit un capteur tactile textile qui comprend une première et une deuxième couche conductrice extérieure et, une troisième couche intermédiaire entre la première et la seconde couche conductrice, ladite troisième couche intermédiaire étant constituée d'un textile non conducteur enduit d'un matériau piézo-résistif. Les première et deuxième couches conductrices extérieures consistent dans un tissu polyester enduites de polypyrrol par exemple.

Le document WO 2009/023937 (D3) décrit quant à lui un système et un vêtement qui incorpore des détecteurs de mesure ou de surveillance de la pression ou de force qui s'exerce sur les pieds, sur des moignons équipés de prothèse ou sur tout autre partie du corps soumise à des forces dues à la situation. Ce document décrit notamment une chaussette sur laquelle au moins une section de tissu de la chaussette est enduite d'un polymère conducteur sur laquelle des fils conducteurs en argent sont cousus.

On connaît également le document WO 01/75778 qui décrit un tissu convenant comme détecteur de pression, comme commutateur ou autre détecteur, comprenant, par construction, un premier conducteur électrique de forme allongée coupant un second conducteur électrique de forme allongée, ledit tissu pouvant être tissé, tricoté, non tissé ou natté. En leur point d'intersection, ces conducteurs sont normalement tenus écartés l'un de l'autre par un intervalle d'air. Il en résulte que l'application d'une pression sensiblement perpendiculairement au plan du tissu provoque un contact des conducteurs.

De plus, le document US 2002/0194934 décrit un capteur de mesure de la pression comprenant un treillis de fils flexibles. Chaque fil est constitué d'une âme en fil électriquement conducteur recouvert d'une couche d'un matériau piezo-résistif, ledit matériau ayant une résistivité électrique qui varie inversement avec la pression exercée sur le matériau.

Enfin, le document WO01/88935 décrit un capteur de pression comprenant une feuille de tissu supportant des granules QTC (« Quantum Tunnelling Composite » en anglais) nickel / silicone incorporées par délayage en suspension aqueuse.

Ces capteurs présentent l'inconvénient de perdre de leur efficacité de mesure au cours du temps, et plus précisément au cours des lavages successifs des vêtements ou similaires. En effet, les couches conductrices sont déposées sur le tissus ou similaire par enduction. Or des lavages successifs altèrent ces couches conductrices rendant les mesures moins précises. Par ailleurs, de tels capteurs de pression seraient immédiatement détériorés en cas de nettoyage à haute température pour stériliser le vêtement et ses capteurs, stérilisation indispensable pour un usage de ces capteurs de pression dans le domaine hospitalier.

Ces capteurs présentent aussi l'inconvénient de ne pas pouvoir utiliser des moyens de productions autorisant une production et des coûts compatibles avec une large distribution. Les moyens de fabrication mis en oeuvre nécessitent un grand nombre d'opérations manuelles qui réduisent le champ d'application à des manipulations de laboratoire.

### BREVE DESCRIPTION DE L'INVENTION

L'un des buts de l'invention est donc de remédier à ces inconvénients en proposant un dispositif de mesure de la distribution de la pression sur une surface souple ou pliable telle qu'un tissu par exemple, de conception simple, peu onéreuse, procurant une mesure de la pression indépendante des phénomènes ambiants tels que la température et/ou humidité régnant à la surface du tissu et procurant une bonne résistance aux lavages fréquents du tissu.

A cet effet et conformément à l'invention, il est proposé un capteur de pression apte à être connecté à un circuit électronique mesurant la variation de résistance électrique lorsqu'une pression est exercée sur le capteur, la pression étant une fonction de la variation de la résistance, le capteur de pression comportant au moins trois couches empilées incluant :
- une couche piézo-résistive,
- une première couche isolante, obtenue à partir d'un arrangement de fibres isolantes, comportant au moins une rangée d'au moins un fil conducteur en contact avec une première face de la couche piézo-résistive, et
- une seconde couche isolante, également obtenu à partir d'un arrangement de fibres isolantes, comportant au moins une rangée d'au moins un fil conducteur, en contact avec la face opposée de la couche piézo-résistive,
caractérisé en ce que la couche piézo-résistive consiste en une pièce de tissu fabriquée à partir de fibres élaborées à partir d'un matériau piézo-résistif..

De préférence, la couche piézo-résistive est obtenue par tricotage, tissage ou similaire de fibres élaborées à partir d'un matériau piézo-résistif.

De manière avantageuse, la couche piézo-résistive comporte des zones piézo-résistives et des zones isolantes.

De préférence, les couches isolantes sont obtenues par tricotage, tissage ou similaire de fibres élaborées à partir d'un matériau isolant.

Par ailleurs, les fils conducteurs de la première couche isolante en contact avec la première face de la couche piézo-résistive sont croisés avec les fils conducteurs de la seconde couche isolante en contact avec la face opposée de la couche piézo-résistive.

Lesdits fils conducteurs de la première couche isolante en contact avec la première face de la couche piézo-résistive s'étendent perpendiculairement aux fils conducteurs de la seconde couche isolante en contact avec la face opposée de la couche piézo-résistive.

De plus, le matériau piézo-résistif consiste dans un polymère conducteur intrinsèque (PCI) et/ou un métal organique et de préférence dans de la polyaniline et/ou dans du polypyrrole et/ou dans des nanotubes de carbone.

Lesdits fils conducteurs consistent dans des fils d'argent et/ou de nickel.

Un autre objet de l'invention concerne un dispositif de mesure de la pression exercée en différents points d'un tissu souple, pliable et/ou extensible et apte à être porté comme vêtement, parement ou similaire, caractérisé en ce qu'il comporte :
- un capteur de pression tel que mentionné ci-dessus, et
- un circuit électronique apte à mesurer la variation de résistance électrique lorsqu'une pression est exercée sur le tissu, la pression étant une fonction de la variation de la résistance.

Par ailleurs, le circuit électrique comporte des moyens de mesure de la variation de la résistance électrique à partir du balayage de la matrice des capteurs, en considérant les fils conducteurs de la couche isolante supérieure en contact avec une face de la couche piézo-résistive et les fils conducteurs de la couche isolante inférieure en contact avec l'autre face de la couche piézo-résistive, le balayage étant obtenu à partir de la sélection séquentielle d'un fil conducteur de la couche supérieure et de la lecture séquentielle d'un fil conducteur de la couche inférieure croisant le fil conducteur de la couche supérieure, la lecture de la variation de la résistance du capteur étant obtenue à partir d'un convertisseur analogique-numérique.

Un dernier objet de l'invention concerne un procédé de fabrication d'au moins un capteur de pression apte à être connecté à un circuit électronique mesurant la variation de résistance électrique lorsqu'une pression est exercée sur le capteur, la pression étant une fonction de la variation de la résistance ; ledit procédé est remarquable en ce qu'il comporte au moins les étapes suivantes de :
- réalisation d'une première couche isolante, obtenue à partir d'un arrangement de fibres isolantes et comportant au moins une rangée d'au moins un fil conducteur,
- réalisation d'une couche piézo-résistive,
- réalisation d'une seconde couche isolante, également obtenu à partir d'un arrangement de fibres isolantes et comportant au moins une rangée d'au moins un fil conducteur, et
- assemblage des deux couches isolantes et de la couche piézo-résistive de telle manière que la ou les rangées de fils conducteurs de la première couche isolante soit en contact avec une première face de la couche piézo-résistive et que la ou les rangées de fils conducteurs de la seconde couche isolante soit en contact avec la face opposée de ladite couche piézo-résistive,
caractérisé en ce que la couche piézo-résistive est fabriquée sous forme d'une pièce de tissu à partir de fibres élaborées à partir d'un matériau piézo-résistif.

L'étape d'assemblage des couches isolantes et de la couche piézo-résistive consiste dans un collage desdites couches.

Alternativement, les couches isolantes et la couche piézo-résistive sont réalisées simultanément par un tricotage ou un tissage en 3 dimensions, la couche piézo-résistive étant constituée par des fils dits espaceurs reliant les deux couches isolantes au niveau des fils conducteurs des couches isolantes.

Selon une autre variante d'exécution du procédé suivant l'invention, au moins une des couches isolantes est tricotée à partir d'un arrangement de fibres isolantes en laissant des rangées vides, puis une troisième couche isolante comportant des rangées de fils conducteurs dont l'espacement est sensiblement égal à l'espacement entre les rangées vides de la couche isolante est assemblée avec ladite couche isolante de telle manière que les fils conducteurs s'étendent dans les rangées vides et soient en contact avec la couche piézo-résistive.

### BREVE DESCRIPTION DES DESSINS

D'autres avantages et caractéristiques ressortiront mieux de la description qui va suivre de plusieurs variantes d'exécution, données à titre d'exemples non limitatifs, du dispositif de mesure de la pression conforme à l'invention, en référence aux dessins annexés, sur lesquels :
- la figure 1 est une représentation schématique en perspective du dispositif de mesure de la pression conforme à l'invention,
- la figure 2 est une représentation schématique en perspective des capteurs du dispositif de mesure de la pression conforme à l'invention,
- la figure 3 est une représentation schématique du circuit électrique du dispositif de mesure de la pression conforme à l'invention représentée sur les figures 1 et 2,
- la figure 4 est une représentation schématique en perspective d'une variante d'exécution du dispositif de mesure de la pression conforme à l'invention,
- la figure 5 est une représentation schématique en perspective d'une seconde variante d'exécution du dispositif de mesure de la pression conforme à l'invention,
- la figure 6 est une représentation schématique des différentes étapes de fabrication de la seconde variante d'exécution du dispositif de mesure de la pression conforme à l'invention représentée sur la figure 5.

### DESCRIPTION DETAILLEE DE L'INVENTION

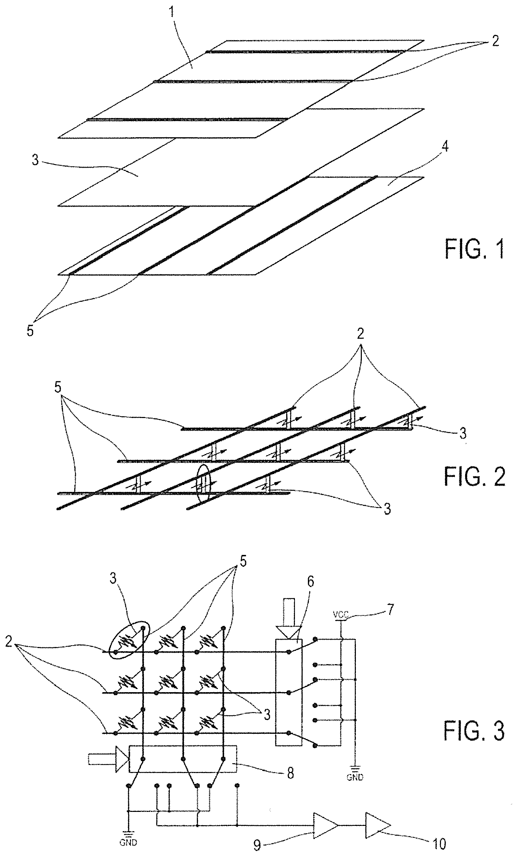

En référence aux figure 1 à 3, le dispositif de mesure de la pression conforme à l'invention est constitué d'au moins trois pièces de tissu empilées, une première pièce de tissu isolant 1, obtenu dans du coton, du nylon ou toute autre matériau souple isolant, comportant des rangées de fils conducteurs 2, solidaires d'une première face d'une pièce de tissu dite piézo-résistive 3, et une seconde pièce de tissu isolant 4, également obtenu dans du coton, du nylon ou toute autre matériau souple isolant, comportant des rangées de fils conducteurs 5, solidaires de la face opposée de la pièce de tissu piézo-résistive 3. La pièce de tissu piézo-résistive 3 consiste par exemple en une pièce de tissu fabriquée à partir de fibres élaborées à partir d'un matériau piézo-résistif de préférence un polymère conducteur intrinsèque (PCI) et/ou métal organique tel que de la polyaniline (PANI) commercialisé par la société ORMECON™ par exemple et/ou du polypyrrole (PPY), par exemple, et/ou des nanotubes de carbone. Ladite pièce de tissu piézo-résistive 3 peut être obtenue par tricotage, tissage ou similaire ou par enduction ou par projection d'un matériau piézo-résistif ou par tout autre procédé bien connu de l'homme du métier. De la même manière, les pièces de tissu isolant 1 et 4 peuvent être obtenues par tricotage, tissage, tressage ou similaire de fibres élaborées à partir d'un matériau isolant.

Lesdits fils conducteurs 2 de la première pièce de tissu isolant 1 en contact avec une première face du tissu piézo-résistif 3 s'étendent sensiblement perpendiculairement aux fils conducteurs 5 de la seconde pièce de tissu isolant 4 en contact avec la face opposée du tissu piézo-résistif 3 formant ainsi une matrice de capteurs de pression.

On observera que les fils conducteurs 2 de la première pièce de tissu isolant 1 en contact avec la première face du tissu piézo-résistif 3 pourront s'étendre suivant un angle quelconque avec les fils conducteurs 5 de la seconde pièce de tissu isolant 4 en contact avec la face opposée du tissu piézo-résistif 3, l'essentiel étant que lesdits fils conducteurs 2 de la première pièce de tissu isolant 1 et les fils conducteurs 5 de la seconde pièce de tissu isolant 4 soient croisés, sans pour autant sortir du cadre de l'invention.

Par ailleurs, en référence à la figure 3, les fils conducteurs 2 de la première pièce de tissu isolant 1 en contact avec une première face du tissu piézo-résistif 3 sont connectés par tout moyen approprié bien connu de l'homme du métier à un premier bus 6 connecté à une source d'alimentation 7 de telle manière que les fils conducteurs 2 soient sélectivement alimentés. Les fils conducteurs 5 de la seconde pièce de tissu isolant 4 en contact avec la face opposée du tissu piézo-résistif 3 sont connectés par tout moyen approprié à un second bus 8 collectant sélectivement la variation de résistance créée par le matériau piézo-résistif lorsqu'une pression est exercée sur une surface déterminée du tissu. Ledit second bus 8 est connecté à une interface analogique 9 connectée à un convertisseur A/D (Analogique/Digital) 10 pour permettre une exploitation des données mesurées.

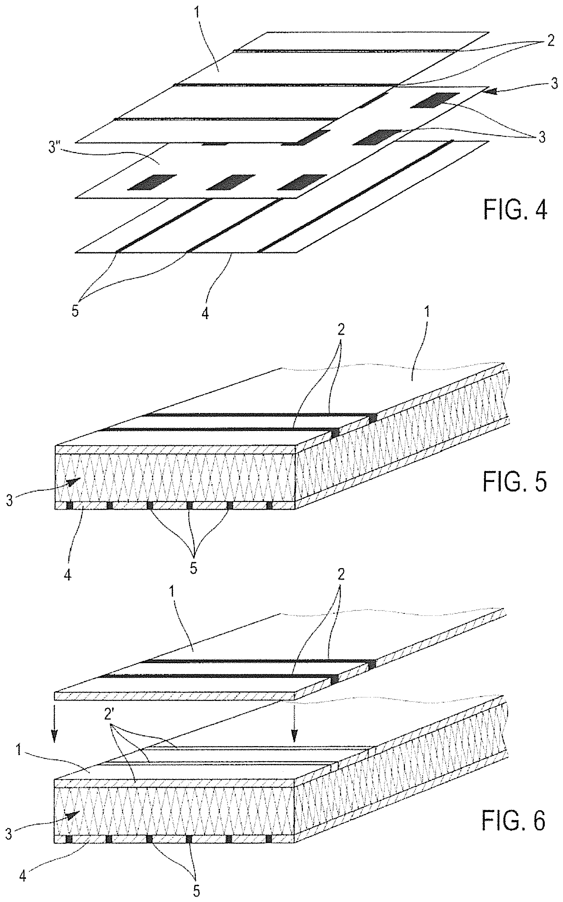

Selon une première variante d'exécution du dispositif suivant l'invention, en référence à la figure 4, ledit dispositif comporte de la même manière que précédemment au moins trois pièces de tissu empilées, une première pièce de tissu isolant 1, obtenu dans du coton, du nylon ou toute autre matériau souple isolant, comportant des rangées de fils conducteurs 2, solidaires d'une première face d'une pièce de tissu dite piézo-résistive 3, et une seconde pièce de tissu isolant 4, également obtenu dans du coton, du nylon ou toute autre matériau souple isolant, comportant des rangées de fils conducteurs 5, solidaires de la face opposée de la pièce de tissu piézo-résistive 3. La pièce de tissu piézo-résistive 3 consiste par exemple en une pièce de tissu fabriquée à partir de fibres élaborées à partir d'un matériau piézo-résistif de préférence un polymère conducteur intrinsèque (PCI) et/ou un métal organique tel que de la polyaniline (PANI) commercialisé par la société ORMECON™ par exemple et/ou du polypyrrole (PPY), par exemple, et/ou dans des nanotubes de carbone. Ladite pièce de tissu piézo-résistive 3 peut être obtenue par tricotage, tissage ou similaire ou par enduction ou par projection d'un matériau piézo-résistif ou par tout autre procédé bien connu de l'homme du métier.

Ce dispositif se distingue du précédent par le fait que la pièce de tissu dite piézo-résistive 3 est également fabriquée à partir de fibres isolantes telles que du coton, du nylon ou similaire, de telle sorte que ladite pièce de tissu piézo-résistive comporte des zones piézo résistives 3' et des zones isolantes.

Lesdits fils conducteurs 2 de la première pièce de tissu isolant 1 en contact avec une première face du tissu piézo-résistif 3 s'étendent sensiblement perpendiculairement aux fils conducteurs 5 de la seconde pièce de tissu isolant 4 en contact avec la face opposée du tissu piézo-résistif 3 au niveau des zones piézo-résistives 3' formant ainsi une matrice de capteurs de pression.

Dans cet exemple particulier de réalisation, les zones piézo-résistives 3' présentent une forme carrée ; toutefois, il est bien évident que lesdites zones piézo-résistives 3' pourront présenter une forme, une taille et un positionnement quelconque sans pour autant sortir du cadre de l'invention.

Selon une seconde variante d'exécution du dispositif suivant l'invention, en référence à la figure 5, ledit dispositif comporte de la même manière que précédemment au moins trois pièces de tissu empilées, une première pièce de tissu isolant 1, obtenu dans du coton, du nylon ou toute autre matériau souple isolant, comportant des rangées de fils conducteurs 2, solidaires d'une première face d'une pièce de tissu dite piézo-résistive 3, et une seconde pièce de tissu isolant 4, également obtenu dans du coton, du nylon ou toute autre matériau souple isolant, comportant des rangées de fils conducteurs 5, solidaires de la face opposée de la pièce de tissu piézo-résistive 3.

Les couches isolantes 1,4 et la couche piézo-résistive 3 sont réalisées simultanément par un tricotage ou un tissage en 3 dimensions (3D), au moyen de toute machine communément appelée métier bien connu de l'homme du métier, les couches isolantes 1,4 formant les couches extérieures du tissu 3D et la couche piézo-résistive 3 étant constituée par des fils dits espaceurs reliant les deux couches isolantes au niveau des fils conducteurs 2,5 des couches isolantes 1,4.

Il est bien évident que le couche piézo-résistive 3 réalisée par un tricotage ou un tissage 3D pourra comporter de la même manière que précédemment des zones piézo-résistive (3'), de formes quelconques telles que de formes carrées par exemple, et des zones isolantes afin de former une matrice de capteurs de pression sans pour autant sortir du cadre de l'invention.

La plupart des métiers permettant la réalisation de tissus 3D n'étant pas adaptés pour la réalisation simultanément de couches isolantes 1,4 comportant des fils conducteurs respectivement dans le sens trame et dans le sens chaîne, le capteur suivant l'invention pourra être réalisé en trois étapes en référence à la figure 6. Dans une première étape, les couches isolantes 1,4 et la couche piézo-résistive 3 sont réalisées simultanément par un tricotage ou un tissage en 3 dimensions, la couche piézo-résistive 3 étant constituée par des fils piézo-résistifs dits espaceurs ou « spacer » reliant les deux couches isolantes 1,4 au niveau des fils conducteurs 2,5 des couches isolantes 1,4. Au moins, l'une des couches isolantes 1 est tricotée à partir d'un arrangement de fibres isolantes en laissant des rangées vides 2', puis, dans une seconde étape, une troisième couche isolante 1' comportant des rangées de fils conducteurs 2 dont l'espacement est sensiblement égal à l'espacement entre les rangées vides 2' de la couche isolante 1 est réalisée. Dans une troisième et dernière étape, la troisième couche isolante l'est assemblée avec ladite couche isolante 1 de telle manière que les fils conducteurs 2 s'étendent dans les rangées vides 2' et soient en contact avec la couche piézo-résistive 3. L'assemblage peut être réalisé par tout moyen approprié bien connu de l'homme du métier tel que par collage par exemple.

Accessoirement, le circuit électrique décrit précédemment pourra avantageusement être connecté à un processeur, tel que le processeur d'un ordinateur de type PC ou similaire, assurant l'analyse des pressions et incluant des méthodes permettant le traitement des applications, et à des moyens permettant d'envoyer les données mesurées à partir des capteurs et le résultat des traitements. Lesdits moyens de transmission des données peuvent consister en tous moyens de transmission filaire ou sans fil tel que des moyens de transmissions wi-fi ®, bluetooth ®, RFID ou similaire, bien connu de l'homme du métier.

On notera que le dispositif suivant l'invention trouvera un grand nombre d'applications pour des capteurs capables de se conformer à différentes formes. Leur souplesse et leur confort permettent une utilisation privilégiée dans la mesure des pressions autour du corps humain. Ils peuvent par exemple être utilisés pour mesurer des pressions excessives qui pourraient entraîner l'apparition de plaies de pression, particulièrement sur des surfaces molles telle que des coussins ou des lits d'hôpitaux ; mais aussi entre le corps et un corset pour les scolioses. Etant donné que ces textiles sensibles s'intègrent facilement aux vêtements, ils peuvent équiper un sous-vêtement ou un vêtement tel que des chaussettes, par exemple, sensibles à la pression qui analysent aussi bien les pressions plantaires que celles exercées autour du pied.

Il va de soi que les couches isolantes 1 et 4 notamment peuvent ne comporter qu'une seule rangée de fils conducteur 2,5 et que chaque rangée peut ne comporter qu'un seul fil conducteur 2,5 sans pour autant sortir du cadre de l'invention.

Enfin, il est bien évident que les exemples que l'on vient de donner ne sont que des illustrations particulières, en aucun cas limitatives quant aux domaines d'application de l'invention.

## Revendications

1. Capteur de pression apte à être connecté à un circuit électronique mesurant la variation de résistance électrique lorsqu'une pression est exercée sur le capteur, la pression étant une fonction de la variation de la résistance, le capteur de pression comportant au moins trois couches empilées incluant :
- une couche piézo-résistive (3),
- une première couche isolante (1), obtenue à partir d'un arrangement de fibres isolantes, comportant au moins une rangée d'au moins un fil conducteur (2) en contact avec une première face de la couche piézo-résistive (3), et
- une seconde couche isolante (4), également obtenu à partir d'un arrangement de fibres isolantes, comportant au moins une rangée d'au moins un fil conducteur (5), en contact avec la face opposée de la couche piézo-résistive (3),
**caractérisé en ce que** la couche piézo-résistive (3) consiste en une pièce de tissu fabriquée à partir de fibres élaborées à partir d'un matériau piézo-résistif.

2. Capteur suivant la revendication 1, **caractérisé en ce que** la couche piézo-résistive (3) comporte des zones piézo-résistives (3') et des zones isolantes.

3. Capteur suivant l'une quelconque des revendications 1 ou 2, **caractérisé en ce que** les fils conducteurs (2) de la première couche isolante (1) en contact avec la première face de la couche piézo-résistive (3) sont croisés avec les fils conducteurs (5) de la seconde couche isolante (4) en contact avec la face opposée de la couche piézo-résistive (3).

4. Capteur suivant la revendication 3, **caractérisé en ce que** les fils conducteurs (11) de la première couche isolante (1) en contact avec la première face de la couche piézo-résistive (3) s'étendent perpendiculairement aux fils conducteurs (5) de la seconde couche isolante (4) en contact avec la face opposée de la couche piézo-résistive (3).

5. Capteur suivant l'une quelconque des revendications 1 à 4, **caractérisé en ce que** le matériau piézo-résistif consiste en un polymère conducteur intrinsèque (PCI), tel que de la polyaniline et/ou du polypyrrole et/ou un métal organique et/ou dans des nanotubes de carbone.

6. Dispositif de mesure de la pression exercée en différents points d'un tissu souple, pliable et/ou extensible et apte à être porté comme vêtement, parement ou similaire, **caractérisé en ce qu'**il comporte :
- un capteur de pression selon l'une des revendications 1 à 5, et
- un circuit électronique apte à mesurer la variation de résistance électrique lorsqu'une pression est exercée sur le tissu, la pression étant une fonction de la variation de la résistance.

7. Dispositif suivant la revendication 6, **caractérisé en ce que** le circuit électrique comporte des moyens adaptés à mesurer la variation de la résistance électrique à partir du balayage de la matrice des capteurs, en considérant les fils conducteurs (2) de la couche isolante supérieure (1) en contact avec une face de la couche piézo-résistive (3) et les fils conducteurs (5) de la couche isolante inférieure (4) en contact avec l'autre face de la couche piézo-résistive (3), le balayage étant obtenu à partir de la sélection séquentielle d'un fil conducteur (2) de la couche supérieure (1) et de la lecture séquentielle d'un fil conducteur (5) de la couche inférieure (4) croisant le fil conducteur (2) de la couche supérieure (1), la lecture de la variation de la résistance du capteur étant obtenue à partir d'un convertisseur analogique-numérique (9).

8. Procédé de fabrication d'au moins un capteur de pression apte à être connecté à un circuit électronique mesurant la variation de résistance électrique lorsqu'une pression est exercée sur le capteur, la pression étant une fonction de la variation de la résistance, le procédé comportant au moins les étapes suivantes de :
- réalisation d'une première couche isolante (1), obtenue à partir d'un arrangement de fibres isolantes et comportant au moins une rangée d'au moins un fil conducteur (2),
- réalisation d'une couche piézo-résistive (3),
- réalisation d'une seconde couche isolante (4), également obtenu à partir d'un arrangement de fibres isolantes et comportant au moins une rangée d'au moins un fil conducteur (5), et
- assemblage des deux couches isolantes (1,4) et de la couche piézo-résistive (3) de telle manière que la ou les rangées de fils conducteurs (2) de la première couche isolante (1) soit en contact avec une première face de la couche piézo-résistive (3) et que la ou les rangées de fils conducteurs (5) de la seconde couche isolante (4) soit en contact avec la face opposée de ladite couche piézo-résistive (3),
**caractérisé en ce que** la couche piézo-résistive (3) est fabriquée sous forme d'une pièce de tissu à partir de fibres élaborées à partir d'un matériau piézo-résistif.

9. Procédé suivant la revendication 8, **caractérisé en ce que** l'étape d'assemblage des couches isolantes (1,4) et de la couche piézo-résistive (3) consiste en un collage desdits couches (1,3,4).

10. Procédé suivant la revendication 8, **caractérisé en ce que** les couches isolantes (1,4) et la couche piézo-résistive (3) sont réalisées simultanément par un tricotage ou un tissage en 3 dimensions, la couche piézo-résistive (3) étant constituée par des fils dits espaceurs reliant les deux couches isolantes au niveau des fils conducteurs (2,5) des couches isolantes (1,4).

11. Procédé suivant la revendication 10, **caractérisé en ce qu'**au moins une des couches isolantes (1,4) est tricotée à partir d'un arrangement de fibres isolantes en laissant au moins une rangée vide, puis une troisième couche isolante comportant au moins une rangée d'au moins un fil conducteur dont l'espacement est sensiblement égal à l'espacement entre les rangées vides de la couche isolante (1,4) est assemblée avec ladite couche isolante (1,4) de telle manière que le ou les fils conducteurs s'étendent dans la ou les rangées vides et soient en contact avec la couche piézo-résistive (3).

## Patentansprüche

1. An eine elektronische Schaltung anschließbarer Drucksensor, der die Veränderung des elektrischen Widerstands misst, wenn auf den Sensor ein Druck ausgeübt wird, wobei der Druck eine Funktion der Widerstandsveränderung ist, wobei der Drucksensor mindestens drei übereinander liegende Schichten umfasst, bestehend aus:
- einer piezoresistiven Schicht (3)
- einer ersten Isolierschicht (1), die aus einer Anordnung aus isolierenden Fasern erhalten wird, die mindestens eine Reihe mit mindestens einem leitenden Draht (2) umfasst, der mit einer ersten Seite einer piezoresistiven Schicht (3) in Kontakt ist, und
- einer zweiten Isolierschicht (4), die ebenfalls aus einer Anordnung aus isolierenden Fasern erhalten wird, die mindestens eine Reihe mit mindestens einem leitenden Draht (5) umfasst, der mit der gegenüberliegenden Seite der piezoresistiven Schicht (3) in Kontakt ist,
**dadurch gekennzeichnet, dass** die piezoresistive Schicht (3) aus einem Stück Gewebe besteht, das aus Fasern hergestellt wird, welche aus einem piezoresistiven Material gebildet werden.

2. Sensor nach Anspruch 1, **dadurch gekennzeichnet, dass** die piezoresistive Schicht (3) piezoresistive Bereiche (3') und isolierende Bereiche umfasst.

3. Sensor nach einem der Ansprüche 1 oder 2, **dadurch gekennzeichnet, dass** die leitenden Drähte (2) der ersten Isolierschicht (1), die mit der ersten Seite der piezoresistiven Schicht (3) in Kontakt sind, mit den leitenden Drähten (5) der zweiten Isolierschicht (4), die mit der gegenüberliegenden Seite der piezoresistiven Schicht (3) in Kontakt sind, verkreuzt sind.

4. Sensor nach Anspruch 3, **dadurch gekennzeichnet, dass** die leitenden Drähte (2) der ersten Isolierschicht (1), die mit der ersten Seite der piezoresistiven Schicht (3) in Kontakt sind, quer zu den leitenden Drähten (5) der zweiten Isolierschicht (4) verlaufen, die mit der gegenüberliegenden Seite der piezoresistiven Schicht (3) in Kontakt sind.

5. Sensor nach einem der Ansprüche 1 bis 4, **dadurch gekennzeichnet, dass** das piezoresistive Material aus einem intrinsisch leitfähigen Polymer (ICP) besteht, wie beispielsweise Polyanilin und/oder Polypyrrol und/oder eine organische Metallverbindung und/oder in Kohlenstoff-Nanoröhren.

6. Vorrichtung zur Messung des Drucks, der an verschiedenen Punkten eines flexiblen, faltbaren und/oder dehnbaren und als Kleidungsstück, Aufschlag oder Entsprechendes tragbaren Gewebes ausgeübt wird, **dadurch gekennzeichnet, dass** die Vorrichtung:
- einen Drucksensor nach einem der Ansprüche 1 bis 5 und
- eine elektronische Schaltung umfasst, die die Veränderung des elektrischen Widerstands bei Ausübung eines Drucks auf den Sensor misst, wobei der Druck eine Funktion der Widerstandsveränderung ist.

7. Vorrichtung nach Anspruch 6, **dadurch gekennzeichnet, dass** die elektrische Schaltung Mittel umfasst, die die Veränderung des elektrischen Widerstands durch Abtasten des Sensorfelds messen können, indem die leitenden Drähte (2) der oberen Isolierschicht (1), die mit einer Seite der piezoresistiven Schicht (3) in Kontakt sind, und die leitenden Drähte (5) der unteren Isolierschicht (4), die mit der anderen Seite der piezoresistiven Schicht (3) in Kontakt sind, berücksichtigt werden, wobei das Abtasten durch sequenzielle Auswahl eines leitenden Drahtes (2) der oberen Schicht (1) und durch sequenzielles Lesen eines leitenden Drahtes (5) der unteren Schicht (4), der mit dem leitenden Draht (2) der oberen Schicht (1) verkreuzt ist, erfolgt, wobei das Lesen der Widerstandsveränderung des Sensors über einen Analog-Digital-Wandler (9) erfolgt.

8. Verfahren zur Herstellung von mindestens einem, an eine elektronische Schaltung anschließbaren Drucksensor, der die Veränderung des elektrischen Widerstands misst, wenn auf den Sensor ein Druck ausgeübt wird, wobei der Druck eine Funktion der Widerstandsveränderung ist, **dadurch gekennzeichnet, dass** das Verfahren mindestens folgende Vorgänge umfasst:
- Herstellung einer ersten Isolierschicht (1), die aus einer Anordnung aus isolierenden Fasern erhalten wird, die mindestens eine Reihe mit mindestens einem leitenden Draht (2) umfasst,
- Herstellung einer piezoresistiven Schicht (3),
- Herstellung einer zweiten Isolierschicht (4), die ebenfalls aus einer Anordnung aus isolierenden Fasern erhalten wird, die mindestens eine Reihe mit mindestens einem leitenden Draht (5) umfasst und
- Verbindung der beiden Isolierschichten (1, 4) und der piezoresistiven Schicht (3), so dass die Reihe(n) aus leitenden Drähten (2) der ersten Isolierschicht (1) mit einer ersten Seite der piezoresistiven Schicht (3) in Kontakt ist/sind und dass die Reihe(n) der leitenden Drähte (5) der zweiten Isolierschicht (4) mit der gegenüberliegenden Seite der piezoresistiven Schicht (3) in Kontakt ist/sind,
**dadurch gekennzeichnet, dass** die piezoresistive Schicht (3) aus einem Stück Gewebe besteht, das aus Fasern hergestellt wird, welche aus einem piezoresistiven Material gebildet werden.

9. Verfahren nach Anspruch 8, **dadurch gekennzeichnet, dass** der Vorgang der Verbindung der Isolierschichten (1, 4) und der piezoresistiven Schicht (3) aus einer Verklebung dieser Schichten (1, 3, 4) besteht.

10. Verfahren nach Anspruch 8, **dadurch gekennzeichnet, dass** die Isolierschichten (1, 4) und die piezoresistive Schicht (3) gleichzeitig durch dreidimensionales Wirken oder Weben hergestellt werden, wobei die piezoresistive Schicht (3) aus sogenannten abstandshaltenden Drähten gebildet wird, die die beiden Isolierschichten im Bereich der leitenden Drähte (2, 5) der Isolierschichten (1, 4) verbinden.

11. Verfahren nach Anspruch 10, **dadurch gekennzeichnet, dass** mindestens eine der Isolierschichten (1, 4) aus einer Anordnung aus isolierenden Fasern gewirkt wird und mindestens eine Reihe frei bleibt, und eine dritte Isolierschicht, die mindestens eine Reihe mit mindestens einem leitenden Draht umfasst, deren Abstand im wesentlichen dem Abstand zwischen den leeren Reihen der Isolierschicht (1, 4) entspricht, derart mit der Isolierschicht (1, 4) verbunden ist, dass der oder die leitende(n) Draht/Drähte in den leere(n) Reihe(n) verlaufen und mit der piezoresistenten Schicht (3) in Kontakt ist/sind.

## Claims

1. Pressure sensor able to be connected to an electronic circuit measuring the variation of electric resistance when a pressure is applied to the sensor, pressure being the function of the resistance variation, the pressure sensor comprising at least three stacked layers including:
- a piezo-resistive layer (3)
- a first insulating layer (1) obtained from an arrangement of insulating fibres, comprising at least one row of at least one conductive yarn (2) in contact with a first surface of the piezo-resistive layer, and
- a second insulating layer (4) also obtained from an arrangement of insulating fibres, comprising at least one row of at least one conductive yarn (5), in contact with the opposite surface of the piezo-resistive layer (3) **characterized in that** the piezo-resistive layer (3) consist of a piece of fabric made of fibres obtained from the piezo-resistive material.

2. The pressure sensor of claim 1, **characterized in that** the piezo-resistive layer (3) comprises piezo-resistive areas (3') and insulating areas.

3. The pressure sensor of any of claims 1 or 2, **characterized in that the** conductive yarns (2) of the first insulating layer (1) in contact with the first surface of the piezo-resistive layer (3) cross with the conductive yarns (5) of the second insulating layer (4) in contact with the opposite surface of the piezo-resistive layer (3).

4. The pressure sensor of claim 3, **characterized in that** the conductive yarns (2) of the first insulating layer (1) in contact with the first surface of the piezo-resistive layer (3) extend perpendicularly to the conductive yarns (5) of the second insulating layer (4) in contact with the opposite surface of the piezo-resistive layer (3).

5. The pressure sensor of any of claims 1 to 4, **characterized in that** the piezo-resistive material is an intrinsically conducting polymer (ICP), such as polyaniline and/or polypyrrole and/or an organic metal, and/or carbon nanotubes.

6. A device for measuring the pressure exerted at different points of a flexible, pliable, and/or extensible fabric capable of being worn as a garment, lapel, or the like, **characterized in that** it comprises:
- a pressure sensor according to one of claims 1 to 5, and
- an electronic circuit able to measure the variation of electrical resistance while the pressure is exerted on the fabric, said pressure being function of the variation of the resistance.

7. The device according to claim 6, **characterized in that** the electric circuit comprises means adapted for measuring the electric resistance variation from the scanning of the sensor array, considering the conductive yarns (2) of the upper insulating layer (1) in contact with a surface of the piezo-resistive layer (3) and the conductive yarns (5) of the lower insulating layer (4) in contact with the other surface of the piezo-resistive layer (3), the scanning being obtained from the sequential selection of a conductive yarn (2) of the upper layer (1) and the sequential reading of a conductive yarn (5) of the lower layer (4) crossing the conductive yarn (2) of the upper layer (1), the reading of the sensor resistance variation being obtained from an analog-to-digital converter (9).

8. A method for manufacturing at least one pressure sensor capable of being connected to an electronic circuit measuring the electric resistance variation when a pressure is exerted on the sensor, the pressure being a function of the resistance variation, it comprises at least the steps of:
- forming a first insulating layer (1) obtained from an arrangement of insulating fibers, comprising at least one row of at least one conductive yarn (2),
- forming a piezo-resistive layer (3),
- forming a second insulating layer (4) also obtained from an arrangement of insulating fibers, comprising at least one row of at least one conductive yarn (5), and
- assembling the two insulating layers (1, 4) and the piezoresistive layer (3) in such a way that the row(s) of conductive yarns (2) of the first insulating layer (1) is (are) in contact with a first surface of the piezoresistive layer (3) and that the row(s) of conductive yarns (5) of the second insulating layer (4) is (are) in contact with the opposite surface of said piezoresistive layer (3),
**characterized in that** the piezo-resistive layer (3) are manufactured in form of a fabric made of fibres obtained from the piezo-resistive material.

9. The method of claim 8, **characterized in that** the step of assembling the insulating layers (1, 4) and the piezo-resistive layer (3) comprises bonding said layers (1, 3,4).

10. The method of claim 8, **characterized in that** the insulating layers (1, 4) and the piezo-resistive layer (3) are simultaneously formed by a 3-dimensional knitting or weaving, the piezo-resistive layer (3) being formed by so-called spacer yarns connecting the two insulating layers at the level of the conductive yarns (2, 5) of the insulating layers (1, 4).

11. The method of claim 10, **characterized in that** at least one of insulating layers (1, 4) is knit from an arrangement of insulating fibers while leaving at least one row empty, after which a third insulating layer comprising at least one row of at least one conductive yarn spaced apart by a distance substantially equal to the spacing between the empty rows of the insulating layer (1, 4) is assembled with said insulating layer (1, 4) so that the conductive yarns extend in the empty rows and are in contact with the piezo-resistive layer (3).
